# EUROPÄISCHE PATENTSCHRIFT

(11) **EP 0 523 619 B1**
(45) Veröffentlichungstag und Bekanntmachung des Hinweises auf die Patenterteilung: **15.03.1995**
(21) Anmeldenummer: 92111968.1
(22) Anmeldetag: 14.07.1992
(51) Int. Cl.: C07C 261/04, C07D 319/06, C07D 317/32

(54) **Verfahren zur Herstellung von N-Cyanimidocarbonaten**
Process for the preparation of N-cyanimidocarbonates
Procédé pour la préparation de N-cyanimidocarbonates

(30) Priorität: 17.07.1991 DE 4123608
(43) Veröffentlichungstag der Anmeldung: 20.01.1993
(73) Patentinhaber: SKW Trostberg Aktiengesellschaft, 83308 Trostberg (DE)
(72) Erfinder: Thalhammer, Franz, Dr., W-8223 Trostberg (DE); Weiss, Stefan, Dr., W-8223 Trostberg (DE)
(74) Vertreter: Böhm, Brigitte, Dipl.-Chem. Dr.

(56) Entgegenhaltungen:
- EP-A- 0 014 064
- DE-A- 3 225 249
- CHEMISCHE BERICHTE Bd. 100, 1967, WEINHEIM DE Seiten 2604 - 2615 E. ALLENSTEIN ET AL 'UEBER DEN N-CYAN-IMIDOKOHLENSAEURE-DIAETHYLESTER UND O-AETHYL-N-CYAN-ISOHARNSTOFFE'

## Beschreibung

Die Erfindung betrifft ein Verfahren zur Herstellung von substituierten N-Cyanimidocarbonaten aus Chlorcyan, Alkoholen und Cyanamid.

Dialkyl-N-cyanimidocarbonate bilden wertvolle Synthesebausteine für substituierte Cyanoguanidin-Verbindungen. Durch sukzessive Substitution der Alkoxygruppen sind auf diese Weise auch unsymmetrisch substituierte Cyanoguanidin-Verbindungen zugänglich, die u.a. als Histamin-2-H Antagonisten Anwendung finden.

Dialkyl-N-cyanimidocarbonate können in vielen Fällen bisher verwendete Reagenzien wie Dimethyl-N-cyanimidodithiocarbonat (J. Org. Chem. Vol. 39, Nr. 11 (1974) 1522 ff) oder Diphenyl-N-cyanimidocarbonat ersetzen, wodurch die Entstehung schwer handhabbarer und/oder gesundheitsgefährdender Nebenprodukte wie Mercaptane oder Phenol vermieden und verbesserte Ausbeuten erhalten werden.

Die Synthese von Diethyl-N-cyanimidocarbonat durch Umsetzung von isoliertem Diethylimidocarbonat mit Cyanamid unter wasserfreien Bedingungen wurde bereits in Chem. Ber. 100, 2604 (1967) beschrieben. Hierbei wurde lediglich eine Ausbeute von 69 % an Rohprodukt erhalten, das zur Reinigung aus absolutem Ether umkristallisiert werden mußte.

In der EP 0 014 064 B1 wird ein Verfahren zur Herstellung von disubstituierten Dimethyl- bzw. Diethyl-N-cyanimidocarbonaten durch Umsetzung eines entsprechend substituierten Imidocarbonats mit Cyanamid in einem Zweiphasensystem, das Wasser und ein mit Wasser nicht mischbares organisches Lösemittel, z. B. Toluol, enthält, beschrieben.

In der DE-OS 32 25 249 wird über ein Verfahren berichtet, wonach zunächst Natriumcyanid unter alkalischen Bedingungen mit dem entsprechenden Alkohol umgesetzt, anschließend Chlor eingeleitet und nach Neutralisation des Reaktionsgemisches und Zugabe von Cyanamid das gebildete substituierte N-Cyanimidocarbonat nach Zugabe von Methylenchlorid aus der organischen Phase gewonnen wird.

Die beschriebenen Verfahren haben den Nachteil, daß bei ihrer Herstellung entweder gesundheitsgefährdende Nebenprodukte entstehen oder organische, z. T. chlorierte, Lösemittel benötigt werden, die einen besonderen Aufwand zu ihrer Beseitigung nach erfolgter Reaktion erfordern.

Es bestand daher die Aufgabe, ein Verfahren zu entwickeln, das die vorstehend genannten Nachteile nicht besitzt und es insbesondere gestattet, substituierte N-Cyanimidocarbonate auch in technischem Maßstab in umweltfreundlicher Weise herzustellen.

Gelöst wurde diese Aufgabe durch das durch die Patentansprüche definierte Verfahren.

Im Gegensatz zu einer früheren Verfahrensbeschreibung ist es heute Stand der Technik, auch Chlorcyan in technischen Mengen sicher zu handhaben und für chemische Reaktionen, z. B. zur Herstellung von Imidocarbonaten, gefahrlos zu verwenden. Man leitet das Chlorcyan vorzugsweise gasförmig in eine durch Natronlauge alkalisch gestellte wäßrige Lösung eines Alkohols ein, hält den pH-Wert der Lösung zwischen 8 und 14 und die Temperatur des Reaktionsgemisches zwischen -5 und 35 °C, bevorzugt zwischen 10 und 20 °C.

Unter diesen Bedingungen reagiert das Chlorcyan schnell und vollständig. Das gebildete Imidocarbonat ist unter diesen Bedingungen überraschenderweise stabiler als im neutralen Milieu.

Für eine Produktion von Imidocarbonaten im technischen Maßstab ist der Einsatz technischen Chlorcyans, wie es z. B. bei der Herstellung von Cyanurchlorid Verwendung findet, aufgrund des erfindungsgemäßen Verfahrens uneingeschräkt möglich. Anstelle gasförmig, kann das Chlorcyan auch in kondensierter oder gelöster Form dem Reaktionsgemisch zudosiert werden.

Das molare Verhältnis von Natronlauge zu Chlorcyan ist abhängig von der Reinheit des verwendeten Chlorcyans. Normalerweise wird ein Verhältnis von Chlorcyan zu Natronlauge von 1 bis 0,75, vorzugsweise 0,9, angewendet.

Die Konzentration der wäßrigen Natronlauge kann zwischen 15 und 50 Gew.-% schwanken; mit besonderem Vorteil verwendet man Natronlauge mit einem Gehalt von 30 Gew.-% Natriumhydroxid.

Das molare Verhältnis für den eingesetzten Alkohol liegt bei 1,9 bis 3,0, vorzugsweise 2,0 bis 2,1 bzw. 1,0 bis 1,5, vorzugsweise 1,1 für Diole, jeweils bezogen auf die eingesetzte Menge Natronlauge.

Als Alkohole zur Herstellung des Imidocarbonats können aliphatische, gerad- oder verzweigtkettige Alkohole mit 1 bis 4 C-Atomen oder Diole mit 2 oder 3 C-Atomen eingesetzt werden.

Die Herstellung des Imidocarbonats erfolgt zweckmäßigerweise in einer Vorrichtung, die geeignet ist, das Chlorcyan in feinster Verteilung über eine geeignete Düse oder einen speziellen Rührer oder mit Hilfe eines statischen Mischers in das Reaktionsgemisch einzubringen.

Eine bevorzugte Vorrichtung zur Herstellung des Dialkylimidocarbonats stellt ein sogenannter Schleifenreaktor dar.

Es hat sich als zweckmäßig erwiesen, die erhaltene alkalische Lösung des Dialkylimidocarbonats zu kühlen und baldmöglich weiter umzusetzen.

Zur Herstellung des Dialkyl-N-cyanimidocarbonats hat es sich überraschenderweise als vorteilhaft herausgestellt, die wäßrige Lösung des Dialkylimidocarbonats der Cyanamidlösung zuzudosieren und nicht umgekehrt die Cyanamidlösung dem Imidocarbonat. Bei gleichzeitiger Zugabe von verdünnter wäßriger Säure hält man den pH-Wert des Reaktionsgemisches im Bereich von 3 bis höchstens 8, bevorzugt zwischen 6 und 7. Auf diese Weise erhält man höhere Gesamtausbeuten als nach den bisher bekannten Verfahren.

Die Konzentration der eingesetzten Cyanamidlösung kann zwischen 20 und 60 Gew.-% schwanken. Bevorzugt ist die Verwendung einer handelsüblichen 50 %igen wäßrigen Lösung. Die molare Menge an Cyanamid bezogen auf Natronlauge beträgt 0,7 bis 1,0, vorzugsweise 0,85, pro Mol-Equivalent Natronlauge.

Als Säuren können ein- oder mehrbasige Mineralsäuren oder Carbonsäuren, wie Salz-, Essig-, Schwefel-, Salpeter- oder Phosphorsäure verwendet werden; vorzugsweise arbeitet man mit 20 %iger Salzsäure.

Als Basen können ein- oder mehrwertige organische oder anorganische Basen verwendet werden; bevorzugt wird Natronlauge in einer Konzentration von 15 bis 50 Gew.-%, vorzugsweise in einer Konzentration von 25 bis 30 Gew.-%, NaOH eingesetzt.

Während der Dosierung der Reaktionspartner oder während einer kurzen Nachreaktionszeit fällt das Dialkyl-N-cyanimidocarbonat aus, die Kristallisation wird durch Kühlung auf unter 0 °C verbessert.

Die Isolierung der Dialkyl-N-cyanimidocarbonate erfolgt durch Zentrifugation oder Filtration bzw. bei öligen Produkten durch Phasentrennung, wonach das Produkt mit kaltem Wasser gewaschen und anschließend getrocknet werden kann.

Die nach dem erfindungsgemäßen Verfahren erhaltenen Dialkyl-N-cyanimidocarbonate erreichen je nach Waschintensität einen Reinheitsgrad zwischen 90 und 99 %. Die Hauptverunreinigung bildet chemisch inertes Natriumchlorid.

Durch Extraktion der Mutterlauge mit organischen Lösemitteln wie Chloroform, Methylenchlorid, Toluol, Essigsäureethylester können ggf. weitere Anteile an Dialkyl-N-cyanimidocarbonat gewonnen werden.

Durch geeignete Wahl der Konzentrationen der Reaktionspartner fällt nur die Menge an wäßriger Mutterlauge an, die zur Lösung der gebildeten Salze nötig ist.

Die folgenden Beispiele dienen zur Erläuterung der Erfindung, ohne sie jedoch darauf zu beschränken.

### Beispiel 1

Ein Gemisch aus 35,2 g (1,10 mol) techn. Methanol und 88,0 g (0,55 mol) einer 25 %igen wäßrigen Natronlauge wurde auf 5 °C abgekühlt und unter externer Kühlung gasförmiges Chlorcyan so eingeleitet, daß die Innentemperatur zwischen 10 und 15 °C gehalten wurde. Bei Erreichen eines pH-Wertes 12,5 wurde die Chlorcyan-Zugabe gestoppt. Es ergab sich ein Verbrauch von 31,4 g (0,51 mol) Chlorcyan. Die so erhaltene Suspension (ein Teil des entstandenen Natriumchlorids fällt aus) wurde unmittelbar danach mit 20 %iger Salzsäure - aber getrennt voneinander - in eine Lösung von 18,9 g (0,45 mol) Cyanamid in 18 ml Wasser gepumpt. Die Zugabegeschwindigkeit beider Komponenten wurde so eingestellt, daß unter Eiskühlung eine Innentemperatur von 20 bis 25 °C eingehalten wurde. Durch Steuerung der Säurezugabe wurde der pH-Wert zwischen 6,4 und 6,7 gehalten. Es ergab sich ein Säureverbrauch von 80,3 g (0,44 mol). Bereits während des Zusammengebens der Reaktanden fiel das Produkt aus. Die Suspension wurde noch 20 Minuten bei 20 °C gerührt und dann eine Stunde lang auf -5 °C gekühlt. Die Kristalle wurden abgesaugt, zweimal mit je 10 ml kaltem Wasser gewaschen und bei Raumtemperatur im Vakuum getrocknet. Es wurden 42,1 g (0,37 mol = 82,0 % d. Th.) farblose Kristalle vom Schmelzpunkt 63,2 °C erhalten. Die Reinheit des erhaltenen Dimethyl-N-cyanimidocarbonats betrug über 99 %. Durch Extraktion von Mutterlauge und Waschwasser mit 100 ml Methylenchlorid ließen sich weitere 5,9 g (0,052 mol) Produkt vom Schmelzpunkt 53 bis 56 °C gewinnen. Die Gesamtausbeute betrug somit 93,3 %.

### Beispiel 2

In eine Mischung aus 21,2 g (0,46 mol) Ethanol und 28,0 g (0,21 mol) 30 %iger wäßriger Natronlauge wurden 11,6 g, (0,19 mol) gasförmiges Chlorcyan so eingeleitet, daß die Temperatur 5 °C nicht überstieg. Beim Erreichen eines pH-Wertes von 12,5 wurde die Zugabe gestoppt und die so erhaltene Diethylimidocarbonat-Lösung unter gleichzeitiger Zugabe von 20 %iger Salzsäure zu 15,1 g (0,18 mol) 50 %iger Cyanamidlösung getropft. Bei äußerer Kühlung wurde dabei eine Temperatur von 15 bis 20 °C eingehalten und die Säurezugabe (insgesamt 28,3 g, 0,16 mol) so gesteuert, daß ein pH-Wert von 6,4 bis 6,6 gehalten wurde. Nach einer Stunde Rühren bei Raumtemperatur wurden die Phasen getrennt und die leicht gelbe organische Phase zweimal mit je 10 ml Wasser ausgeschüttelt. Das Diethyl-N-cyanimidocarbonat fiel als gelbliches Öl mit einer Ausbeute von 15,2 g (0,107 mol = 59 % d. Th.) an.

### Beispiel 3

In ein Gemisch aus 13,6 g (0,22 mol) Ethylenglykol und 33,6 g (0,21 mol) 25 %iger wäßriger Natronlauge wurde unter Eiskühlung gasförmiges Chlorcyan (11,6 g, 0,19 mol) mit solcher Geschwindigkeit eingeleitet, daß die Temperatur 10 °C nicht überstieg. Bei Erreichen eines pH-Wertes von 12,4 wurde die Zugabe gestoppt und noch 15 Minuten bei 10 °C gerührt. Die erhaltene Lösung wurde unter pH-Kontrolle (6,4 bis 6,6) bei 20 bis 25 °C durch gleichzeitige Zugabe von 21,4 g (0,12 mol) 20 %iger wäßriger Salzsäure zu einer vorgelegten wäßrigen handelsüblichen Lösung von 7,6 g (0,18 mol) Cyanamid in 7,5 ml Wasser getropft, 30 Minuten bei Raumtemperatur gerührt und dann auf -5 °C gekühlt. Dabei fielen 9,62 g (85,9 mol = 48 % d. Th.) farblose Kristalle vom Schmelzpunkt 74 bis 77 °C an, die abgesaugt, getrocknet und als 0,0'-Ethylen-N-cyanimidocarbonat identifiziert wurden.

### Beispiel 4

Analog Beispiel 1 wurden 16,0 g (0,5 mol) Methanol, 41,4 g (0,21 mol) 20,3 %ige Natronlauge und 12,3 g (0,20 mol) Chlorcyan eingesetzt. Im zweiten Schritt wurden 14,6 g (0,18 mol) einer 50 %igen Cyanamidlösung vorgelegt und 32,3 g (0,18 mol) 20 %ige Salzsäure verbraucht. Ohne Wäsche mit Wasser fielen 16,6 g Produkt vom Schmelzpunkt 58 bis 62,5 °C und einer Reinheit von 94,6 % an. Der Chloridgehalt betrug 3,0 %. Durch Extraktion der Mutterlauge mit 40 ml Methylenchlorid wurden weitere 3,3 g Produkt mit einem Schmelzpunkt von 56 bis 58 °C und einer Reinheit von 88,8 % erhalten. Die Gesamtreinausbeute betrug 90,4 %, bezogen auf Cyanamid.

### Beispiel 5

1100 g (16,6 mol) Chlorcyan (techn. 93 %) wurden gasförmig in eine Lösung von 1216 g (38,0 mol) Methanol (techn.) und 2857 g (20,0 mol) 28,7 %ige Natronlauge so eingeleitet, daß die Temperatur zwischen 10 und 20 °C gehalten wurde. Die Zufuhr des Chlorcyans wurde bei Erreichen eines pH-Wertes von 12,0 gestoppt. Diese Lösung wurde so zu 1260 g (15,0 mol) einer vorgelegten 50 %igen Cyanamidlösung gepumpt, daß bei gleichzeitiger Zugabe von 2762 g (14,0 mol) 18 %iger Salzsäure die Temperatur durch Kühlung bei 20 bis 25 °C und der pH-Wert bei 6,4 bis 6,6 gehalten wurde. Die erhaltene Suspension wurde auf 0 °C gekühlt, abgesaugt und der Filterkuchen zweimal mit 1000 ml kaltem Wasser gewaschen. Nach Trocknung bei 35 °C im Vakuum (100 mbar) wurden 1426 g farbloses Produkt mit einem Schmelzpunkt von 61 bis 63 °C erhalten. Die Analyse ergab eine Reinheit von 99,4 %, der Chloridgehalt lag bei 0,2 %.

### Bespiel 6

17,7 g (0,29 mol) Chlorcyan wurden bei -3 °C kondensiert und unter Kühlung (10 bis 15 °C) zu einer Lösung von 40,0 g (0,30 mol) einer 30 %igen Natronlauge und 18,4 g (0,58 mol) Methanol getropft. Hierbei stellte sich ein pH-Wert von 11,8 ein. Die erhaltene Lösung des Dimethylimidocarbonats wurde gleichzeitig mit 37 g (0,2 mol) einer 20 %igen Salzsäure 19,3 g (0,23 mol) einer 50 %igen Cyanamidlösung in der Weise zugetropft, daß die Temperatur des Reaktionsgemisches 20 °C nicht überstieg und der pH-Wert zwischen 5,9 und 6,6 lag. Das ausgefallene Produkt wurde nach 1 1/2 stündigem Kühlen auf 0 °C abgesaugt und ohne Waschen getrocknet. Dabei fielen 25,5 g Kristalle vom Schmelzpunkt 52 bis 55 °C und einer Reinheit von 93,8 % an. Die Ausbeute an reinem Produkt beträgt 91,3 %.

## Patentansprüche

1. Verfahren zur Herstellung von N-Cyanimidocarbonaten der Formel 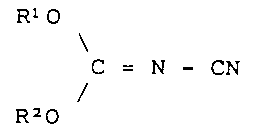 worin R¹ und R² gleich sind und Alkylreste mit 1 bis 4 Kohlenstoffatomen bedeuten oder R¹ und R² miteinander verbunden sind, so daß eine, ggf. durch einen Alkylrest mit 1 bis 3 Kohlenstoffatomen substituierte Ethylen- oder Propylenkette entsteht, dadurch gekennzeichnet, daß man das in alkalischwäßriger Lösung aus dem entsprechenden Alkohol und Chlorcyan erhaltene Imidocarbonat mit einer Säure zu einer vorgelegten wäßrigen Cyanamidlösung in der Weise zugibt, daß das Reaktionsgemisch einen pH-Wert von 3 bis 8 aufweist.

2. Verfahren nach Anspruch 1, dadurch gekennzeichnet, daß man die Reaktion zwischen dem Alkohol und Chlorcyan in wäßrig-alkalischer Lösung zwischen -5 und 35 °C, bevorzugt zwischen 10 und 20 °C, durchführt und den pH-Wert der Lösung zwischen 8 und 14 hält.

3. Verfahren nach den Ansprüchen 1 und 2, dadurch gekennzeichnet, daß man das in Lösung befindliche Imidocarbonat unmittelbar nach seiner Herstellung gleichzeitig mit wäßriger Salzsäure in der Weise einer wäßrigen Cyanamidlösung zudosiert, daß die Temperatur des Reaktionsgemisches sich in den Grenzen von 10 bis 30 °C, vorzugsweise bei etwa 20 °C, bewegt und ein pH-Bereich von 3 bis 8, vorzugsweise zwischen 6 und 7, eingehalten wird.

4. Verfahren nach den Ansprüchen 1 bis 3, dadurch gekennzeichnet, daß R¹ und R² Methyl- oder Ethylreste sind.

5. Verfahren nach den Ansprüchen 1 bis 3, dadurch gekennzeichnet, daß R¹ und R² zusammen eine Ethylenkette bedeuten.

6. Verfahren nach den Ansprüchen 1 bis 5, dadurch gekennzeichnet, daß die Menge an Chlorcyan 0,75 bis 1 , vorzugsweise 0,9 Mol, pro Mol-Equivalent Base beträgt.

7. Verfahren nach den Ansprüchen 1 bis 6, dadurch gekennzeichnet, daß man gasförmiges Chlorcyan verwendet.

8. Verfahren nach den Ansprüchen 1 bis 7, dadurch gekennzeichnet, daß die molare Menge für den eingesetzten Alkohol 1,9 bis 3,0, vorzugsweise 2,0 bis 2,1 bzw. 1,0 bis 1,5, vorzugsweise 1,1 für Diole, bezogen auf das eingesetzte Mol-Equivalent Base, beträgt.

9. Verfahren nach den Ansprüchen 1 bis 8, dadurch gekennzeichnet, daß man als Cyanamidkomponente handelsübliche, 20 bis 60 %ige wäßrige Cyanamidlösung in einer Menge von 0,7 bis 1 Mol, vorzugsweise 0,85 Mol, pro Mol-Equivalent Base verwendet.

10. Verfahren nach den Ansprüchen 1 bis 9, dadurch gekennzeichnet, daß man als Säure 10 bis 35 %ige Salzsäure verwendet.

11. Verfahren nach den Ansprüchen 1 bis 10, dadurch gekennzeichnet, daß man als Base Natronlauge in einer Konzentration von 15 bis 50 Gew.-%, vorzugsweise 25 bis 30 Gew.-%, verwendet.

12. Verfahren nach den Ansprüchen 1 bis 11, dadurch gekennzeichnet, daß man das N-Cyanimidocarbonat aus der wäßrigen Mutterlauge durch Abkühlen unter 0 °C auskristallisiert und durch übliche Verfahren isoliert.

13. Verfahren nach Anspruch 12, dadurch gekennzeichnet, daß man weiteres Produkt durch Extraktion der Mutterlauge mit organischen Lösemitteln, vorzugsweise Methylenchlorid, erhält.

## Claims

1. Process for the preparation of N-cyanoimidocarbonates of the formula:- 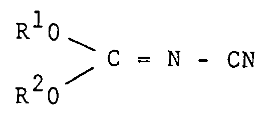 wherein R¹ and R² are the same and signify alkyl radicals with 1 to 4 carbon atoms or R¹ and R² are joined together so that an ethylene or propylene chain results possibly substituted by an alkyl radical with 1 to 3 carbon atoms, characterised in that one adds the imidocarbonate obtained in alkaline aqueous solution from the appropriate alcohol and cyanogen chloride with an acid to an aqueous cyanamide solution in such a manner that the reaction mixture has a pH value of 3 to 8.

2. Process according to claim 1, characterised in that one carries out the reaction between the alcohol and cyanogen chloride in aqueous alkaline solution between -5 and 35°C, preferably between 10 and 20°C, and maintains the pH value of the solution between 8 and 14.

3. Process according to claims 1 and 2, characterised in that one doses the imidocarbonate present in solution, immediately after its preparation, simultaneously with aqueous hydrochloric acid into an aqueous cyanamide solution in such a manner that the temperature of the reaction mixture varies within the limits of 10 to 30°C, preferably at about 20°C, and a pH range of 3 to 8, preferably between 6 and 7, is maintained.

4. Process according to claims 1 to 3, characterised in that R¹ and R² are methyl or ethyl radicals.

5. Process according to claims 1 to 3, characterised in that R¹ and R² together signify an ethylene chain.

6. Process according to claims 1 to 5, characterised in that the amount of cyanogen chloride amounts to 0.75 to 1, preferably 0.9 mol per mol equivalent of base.

7. Process according to claims 1 to 6, characterised in that one uses gaseous cyanogen chloride.

8. Process according to claims 1 to 7, characterised in that the molar amount for alcohol used amounts to 1.9 to 3.0, preferably to 2.0 to 2.1, or to 1.0 to 1.5, preferably to 1.1 for diols, referred to mol equivalent of base used.

9. Process according to claims 1 to 8, characterised in that, as cyanamide component, one uses commercially-avail able 20 to 60% aqueous cyanamide solution in an amount of 0.7 to 1 mol, preferably 0.85 mol, per mol equivalent of base.

10. Process according to claims 1 to 9, characterised in that one uses 10 to 35% hydrochloric acid as acid.

11. Process according to claims 1 to 10, characterised in that, as base, one uses caustic soda solution in a concentration of 15 to 50 wt.%, preferably of 25 to 30 wt.%.

12. Process according to claims 1 to 11, characterised in that one crystallises the N-cyanoimidocarbonate from the aqueous mother liquor by cooling below 0°C and isolates by conventional processes.

13. Process according to claim 12, characterised in that one obtains further product by extraction of the mother liquor with organic solvents, preferably methylene chloride.

## Revendications

1. Procédé pour la préparation de N-cyanimidocarbonates de la formule 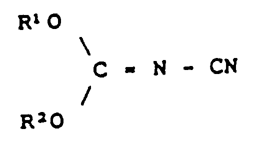 dans laquelle R¹ et R² sont identiques et signifient des radicaux alkyle avec 1 à 4 atomes de carbone où R¹ et R² sont liés ensemble de façon à créer une chaîne éthylène ou propylène éventuellement substituée par un radical alkyle avec 1 à 3 atomes de carbone, caractérisé en ce que l'on ajoute l'imidocarbonate obtenu en solution aqueuse alcaline à partir de l'alcool correspondant et du chlorocyan avec un acide à une solution de cyanamide aqueuse introduite de façon que le mélange réactionnel présente une valeur du Ph de 3 à 8.

2. Procédé selon la revendication 1, caractérisé en ce que l'on conduit la réaction entre l'alcool et le chlorocyan en solution alcacine aqueuse entre - 5 et 35°C, de préférence entre 10 et 20°C, et on maintient la valeur du pH de la solution entre 8 et 14.

3. Procédé selon les revendications 1 et 2, caractérisé en ce que l'on ajoute, de façon dosée, l'imidocarbonate se trouvant dans la solution directement après sa fabrication, simultanément avec de l'acide chlorhydrique aqueux, a une solution de cyanamide aqueuse de façon à maintenir la température du mélange réactionnel dans les limites de 10 à 30°C, de préférence aux alentours de 20°C, et on maintient la plage du pH de 3 à 8, de préférence entre 6 et 7.

4. Procédé selon les revendications 1 à 3, caractérisé en ce que R¹ et R² sont des radicaux méthyle ou éthyle.

5. Procédé selon les revendications 1 à 3, caractérisé en ce que R¹ et R² signifient conjointement une chaîne éthylène.

6. Procédé selon les revendications 1 à 5, caractérisé en ce que la quantité de chlorocyan est de 0,75 à 1, de préférence 0,9 mole par mole équivalent de base.

7. Procédé selon les revendications 1 à 6, caractérisé en ce que l'on utilise du chlorocyan gazeux.

8. Procédé selon les revendications 1 à 7, caractérisé en ce que la quantité molaire pour l'alcool mis en oeuvre est de 1,9 à 3,0, de préférence 2,0 à 2,1 ou 1,0 à 1,5, de préférence 1,1 pour les dioles rapportées au mole équivalent base mis en oeuvre.

9. Procédé selon les revendications 1 à 8, caractérisé en ce que l'on utilise en tant que composant cyanamide une solution de cyanamide aqueuse du commerce à 20 jusqu'à 60 % dans une quantité de 0,7 à 1 mole, de préférence 0,85 mole par mole-équivalent de base.

10. Procédé selon les revendications 1 à 9, caractérisé en ce que l'acide utilisé est l'acide chlorhydrique à 10 jusqu'à 35 %.

11. Procédé selon les revendications 1 à 10, caractérisé en ce que l'on utilise comme base la lessive de soude dans une concentration de 15 à 50 % en poids, de préférence 25 à 30 % en poids.

12. Procédé selon les revendications 1 à 11, caractérisé en ce qu'on recristallise le N-cyanamidocarbonate sur la lessive mère aqueuse par refroidissement au-dessous de 0°C et que l'on isole par procédé habituel.

13. Procédé selon la revendication 12, caractérisé en ce que l'on obtient un produit supplémentaire par extraction de la lessive mère avec des solvants organiques, de préférence du chlorure de méthylène.
